# EUROPEAN PATENT SPECIFICATION

(11) **EP 1 042 024 B1**
(45) Date of publication and mention of the grant of the patent: **10.11.2004**
(21) Application number: 97954463.2
(22) Date of filing: 23.12.1997
(51) Int. Cl.: A61M 15/00

(54) **PARTICLE SEPARATOR FOR AN INHALER**
TEILCHENABSCHEIDER FÜR EINEN INHALATOR
SEPARATEUR DE PARTICULES POUR INHALATEUR

(43) Date of publication of application: 11.10.2000
(73) Proprietor: Euro-Celtique S.A., 2330 Luxembourg (LU)
(72) Inventor: FLEISCHER, Wolfgang, D-55218 Ingelheim (DE)
(74) Representative: Hilleringmann, Jochen, Dipl.-Ing.
(86) International application number: PCT/EP1997/007265
(87) International publication number: WO 1999/033505

(56) References cited:
- EP-A- 0 237 507
- EP-A- 0 712 637
- WO-A-97/05917
- WO-A-97/25086
- WO-A-97/41910
- US-A- 2 604 094
- US-A- 5 676 130

## Description

The invention relates to a particle separator for separating a particle fraction of an inhalable particle/air flow generated by inspired air, said particle separator being provided particularly to be mounted on a device for generating inhalable particles of an active substance by surface treatment of a solidified medicant supply.

In prophylactic and healing therapies of respiratory tract diseases which can be performed through resorption via the lung, use is made of inhalation devices by which the patient inhales a controlled-dosage aeresol, with or without a propellant gas, along with inhalable particles. For this purpose, the particles are taken in predetermined quantities (dosage) from a supply of a medicant by abrasion/ scraping or by a different manner of detaching the particles, and then enter a carrier gas flow which together with the particles will be inhaled by the patient.

To reduce environmental damage, efforts are made to replace the dosage aeresol generators containing a propellant gas by generators which are free of propellant gas. Thus, presently, for generating the active-substance particles, use is made of powder generators provided to have an air flow passing therethrough which is generated by the patient when inhaling air. This air flow takes along the active-substance particles which have been abraded, scraped or otherwise detached from a medicant supply, so that an air flow containing active-substance particles will enter the oral and pharyngeal regions and the bronchi of the patient. Such inhalators with powder generators are known, for instance, from DE-A 40 27 390, EP-A 0 407 028 and WO-A 93/24165.

For obtaining a good therapeutic effect, it is of advantage if the coarser particles of the flow of active-substance particles/air are retained and the patient will thus inhale primarily the smaller particles. Besides, depending on the respective composition of the active substance of the medicant supply, these coarser particles, which are deposited primarily in the oral and pharyngeal regions of the patient, can lead to uncomfortable side effects so that it is advisable also under this aspect to separate these coarser particles before the flow of active-substance particles/air reaches the oral and pharyngeal regions of the patient.

A particle separator according to the preamble of claim 1 is known from WO-A-97/41910. This particle separator is provided with partition walls arranged perpendicular to the extension of the longitudinal housing between its inlet and outlet. Already this particle separator has relatively good separation characteristics with regard to the larger particle fractions.

Another particle separator used as an inhalation device is known from WO-A-97/25086. This device comprises a housing having a conduit including a plurality of staggered plates. The staggered plates extend into the conduit from opposite sides of the conduit at an angle of less than 90° to the sides of the conduit and are inclined towards the outlet to create a plurality of constrictions within the conduit and a plurality of changes in the direction of the airflow through the conduit.

It is an object of the invention to provide an inhalator device which has a still further improved separation characteristic for coarser particles.

For solving the above object, the invention proposes a particle separator for separating a particle fraction of an inhalable particle/air flow generated by inspired air, said particle separator being provided particularly to be mounted on a device for generating inhalable active-substance particles by surface treatment of a preferably solidified medicant supply and further comprising the features of claim 1. The dependent claims define individual embodiments of the invention.

According to the invention, the carrier gas flowing through the housing of the particle separator is subjected to a plurality of deflections which cause the larger and heavier particles to be carried out of the carrier gas flow because of the large centrifugal force acting on them (fractionating particle separation). To deflect the carrier gas flow, the housing has arranged therein a plurality of partition walls with respectively at least one opening for restricting the cross section of the flow, the openings of respectively adjacent successive partition walls being displaced relative to each other. Particularly, the mutual displacement of the openings is selected in a manner providing for a helical carrier gas flow in the housing between the inlet and the outlet.

Surprisingly, it has been found that the separation characteristic can be increased if the partition walls are arranged at an inclination, particularly at an inclination in opposite directions to each other. In such a configuration, the angle of inclination is in particular substantially 65° to 85°, particularly 75° to 80°, relative to the extension of the housing. Thereby, the gas flow is subjected to a further deflection in addition to the positive deflection already effected by the rotational displacement of adjacent partition walls. Provided are groups of mutually parallel partition walls, with the partition walls of each group having a different angle of inclination. Preferably, there exist two groups of differently inclined partition walls whose angles of inclination are inverse to each other. In other words, the partition walls of these groups are inclined respectively at identical angles but in different directions about a plane extending substantially transversely to the longitudinal direction of the housing. Particularly, adjacent successive partition walls are alternately turned or inclined to present a zig-zag-like arrangement of the partition walls when viewed from the side.

The particle separator according to the invention makes it possible, in a simple constructional manner, to retain and fractionate those particles of a flow of active-substance particles/air generated by air inhaled by the patient which are larger than a maximum allowable size, so that these particles will not enter the oral and pharyngeal regions of the patient. Thus, hardly any larger active-substance particles will become deposited in the oral and pharyngeal regions of the patient, thus reducing the extent of the side effects of the medicant. Instead, it is the smaller active-substance particles which are transported, by the air inhaled by the patient, all the way into the bronchi and lungs where an optimum therapeutic effect is obtained.

The particle separator of the invention can be mounted particularly onto the inhalators according to DE-A 40 27 390, EP-A 0 407 028 and WO-A 93/24165; the subject matter of these printed publications is herewith incorporated by reference into the subject matter of the present application. Tests performed with the inventive particle separator mounted onto the mouthpiece of the inhaltor according to WO-A 93/24165 have shown that - independent from the air flow volume, which as a matter of experience is between 300 cm³ and 1.5 dm³, and independent from the rate of air flow, which as a matter of experience is between 15 and 90 dm³ per minute - active-substance particles from a size of substantially 5.8 µm are separated, whereas the range of particle sizes below substantially 5.8 µm, i.e. particles of a size between 0.5 and substantially 5.8 µm, can be inhaled.

According to an advantageous embodiment of the invention, it is provided that said openings are formed as round holes in the partition walls, with the partition walls limiting the holes on all sides. The size of the portions of the partition walls bordering the openings defines the size of the baffle faces subjected to the onflow of the flow of active-substance particles/air. The displacement of the openings respecticvely to the regions of the partition walls adjacent the housing makes it possible to influence the size of the baffle faces. In a particularly advantageous configuration, the openings are delimited partially by the inner side of the housing, i.e. the openings are formed, as it were, as edge recesses of the partition walls.

To keep the fractionating of particles substantially independent from the tidal volume and the rate of air flow, it is of advantage if - as observed over the whole flow path - the parameters of the flow, i.e. the velocity and/or the cross section of the flow as well as the flow volume, are varied, wherein this variation and these variations, respectively, can be performed regularly and irregularly, respectively, and/or continuously and discontinuously, respectively, and/or steadily and unsteadily, respectively. In this regard it is preferred, that the openings of the partition walls have different sizes from one partition wall to the next partition wall. Preferably, the size of the openings of the partition walls decreases from the inlet to the outlet of the housing, particularly in a uniform manner. Such a change of the openings of the partition walls, which towards the outlet of the housing become ever smaller, causes the flow velocity to increase towards the outlet of the housing. The increase of the velocity results in an increase of the centrifugal forces acting on the particles, so that larger particles are separated in any event before the flow of active-substance particles/air has left the housing.

By way of alternative or in addition to the variation of the size of the openings, it is provided according to another advantageous embodiment of the invention that the distance between respectively adjacent partition walls is different from one partition wall to the next one. In this regard it is preferred, that the distance of the partition walls is suitably reduced from the inlet towards the outlet of the housing, so that the deflections that the flow of active-substance particles/air undergoes within the housing of the particle separator, become ever smaller towards the outlet of the housing. Thereby, it is accomplished to a still higher extent that the larger active-substance particles, which have to be separated, are retained by the partition walls of the separator and thus do not issue from the outlet of the housing.

The ratio between the largest opening and the smallest opening of the partition walls is between 0.5 and 0.05 and particularly between 0.33 and 0.1. The sizes of the openings which are larger than the smallest opening and are smaller than the largest opening, vary in uniform or nonuniform steps. The ratio between the distances of adjacent partition walls is preferably between 1 and 1.5, the ratio between the largest distance and the smallest distance between partition walls ranging from 1 to 10.

Suitably, the housing of the particle absorber comprises two to seven and particularly three to five partition walls, wherein the best results have been obtained using a particle absorber with three partition walls.

Embodiments of the invention will be explained in greater detail hereunder with reference to the drawings. In the drawings -
- Fig. 1: is a perspective sectional view of an inhalator with powder generator for generating a flow of active-substance particles/air during inhalation of air through the inhalator,
- Fig. 2: is a longitudinal sectional view of a first embodiment of a particle-separator attachment unit for the powder generator of the inhalator according to Fig. 1, and
- Figs. 3 to 8: are sectional views of the housing of the particle separator according to Fig. 2, showing the partition walls of the separator and the size and arrangement of the openings in the flow direction, and
- Figs. 9 and 10: are a longitudinal sectional view and an open side view, respectively, of second and thirds embodiment of a particle-separator attachment unit for the powder generator of the inhalator according to Fig. 1.

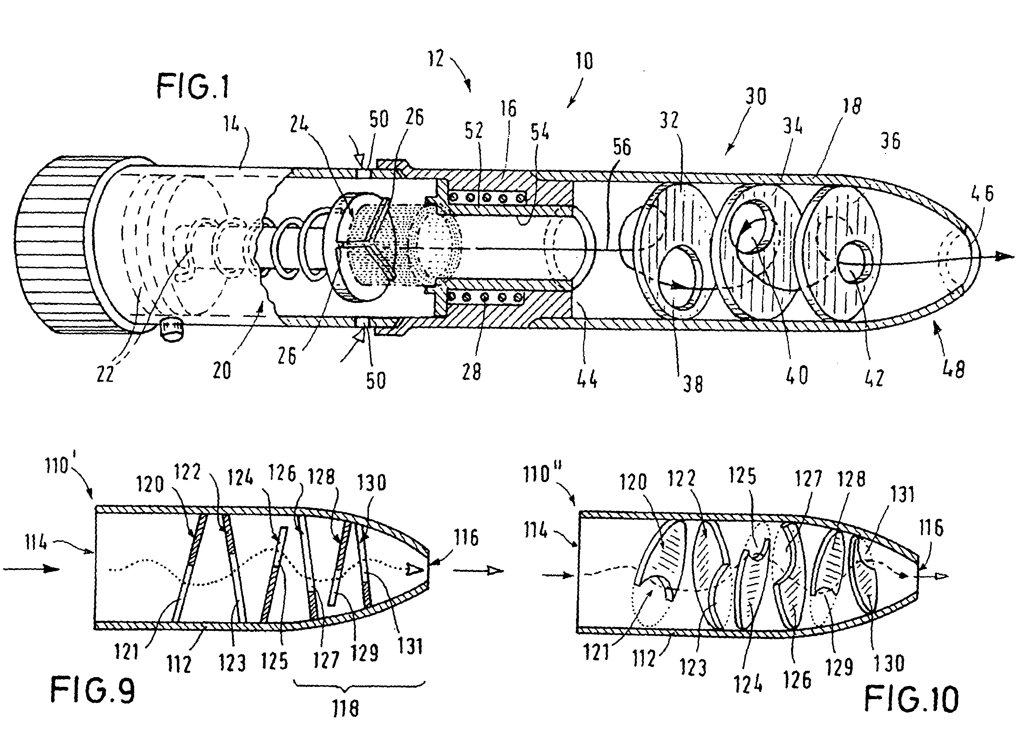

Before starting the description of Fig. 1, it is to be noted that this Figure merely serves for the general description and explantion of an inhalator according to the state of the art (WO-A-97/41910). The invention, i.e. the inclined arrangement of the partition walls arranged at a mutual rotational displacement, is not shown in Fig. 1.

As shown in Fig. 1, the inhalator 10 comprises a three-part housing 12 consisting of three housing portions 14, 16 and 18 adapted to be mounted to each other. In housing portion 14, an automatic release device 20 is arranged for the dosed release of active-substance particles of a supply of a medicant. The release device 20 comprises a mechanical spring-wound motor 22 which can be wound up by hand and is arranged to rotate a end-milling cutter 24. The end-milling cutter 24 comprises cutting tools 25 abutting one end face of an annular solidified supply of a medicant 26. This medicant supply 26 is arranged in the central housing portion 16 of housing 12 and is pressed against the end-milling cutter 24 by a pressing spring 28. Mounted on the central housing portion 16 is the housing portion 18, forming part of a particle separator 30. Within the housing portion 18 (hereunder briefly referred to as "housing"), a plurality - in the instant embodiment, three - of partition or baffle walls 32,34,36 are arranged, each of them having an opening 38,40,42 formed therein. The housing 18 of particle separator 30 is further provided with an inlet 44 and an outlet 46 opposite thereto. In that region where the partition walls 32-36 are arranged within the housing, the housing 18 extends substantially in a cylindrical shape, whereas, starting from the partition wall closest to outlet 46 up to the outlet 46, the housing 18 has a conical shape forming a mouthpiece 48.

As evident from Fig. 1, the housing portion 14 containing the release device 20 is provided with air inlet openings 50 arranged level with the end-milling cutter 24. Between the air inlet openings 50 and the air outlet 46 of the particle separator, which air outlet 46 is also the air outlet of the inhalator 10, an air flow is generated when a patient inhales air through the air outlet 46. The air flow entering through the air inlet openings 50 passes through an intermediate space between the end-milling cutter 24 and the end face of medicant supply 26 from which particles are released by the action of the cutting tools 25 of end-milling cutter 24. From there, the air flow passes axially through the annular medicant supply and further through a press-on shell 52 which is in abutment on the medicant supply 26 and is pressed towards the end-milling cutter 24 by the spring 28 supported on a shoulder 54 of the central housing portion 16. The housing 18 of particle separator 30 is mounted to the central housing portion 16 so that the air leaving the central housing portion 16 enters the housing 18 through the inlet 44 of housing 18. As can be seen in Fig. 1, the openings 38, 40 and 42 of the partition walls 32,34,36 located behind each other in the flow direction 56, are arranged in a mutually displaced relationship, i.e. are arranged at positions rotated relative to each other about the longitudinal axis of housing 18. By this arrangement, the flow 46 is subjected to non-uniform deflections and thereby is caused to move in a substantially helical manner, thus following a curvilinear path. The openings 38-42 are reduced in size towards outlet 46; in other words, the opening 38 of the partition wall 32 of housing 18 closest to inlet 44 is largest, while the partition wall 36 closest to outlet 46 comprises the smallest opening 42. Further, the distance between adjacent partition walls varies towards outlet 46 of housing 18, which is also evident from Fig. 1. This means that the distance between the partition wall 32 facing towards the inlet 44 of housing 18 and the central partition wall 34, is larger than the distance between the central partition wall 34 and the partition wall 36 facing towards the outlet 46. The decrease of the size of the openings towards outlet 46 results in an increase of the flow velocity towards outlet 46. The reduction of the distances between adjacent partition walls 32,34,36 has the effect that the deflections that the flow is subjected to become narrower towards outlet 46. Both dimensional changes, i.e. the changes of the sizes of the openings and the changes of distances between the partition walls, lead to an optimum fractionating of the particles, which during rotation of the end-milling cutter 24 are removed from the medicant supply 26 and upon simultaneous suctional intake of air are taken along by the air flow. Notably, the centrifugal forces acting on the particles increase towards the outlet 46 of inhalator 10 because, on the one hand, the flow velocity is increased and, on the other hand, the flow is subjected to ever narrower deflections.

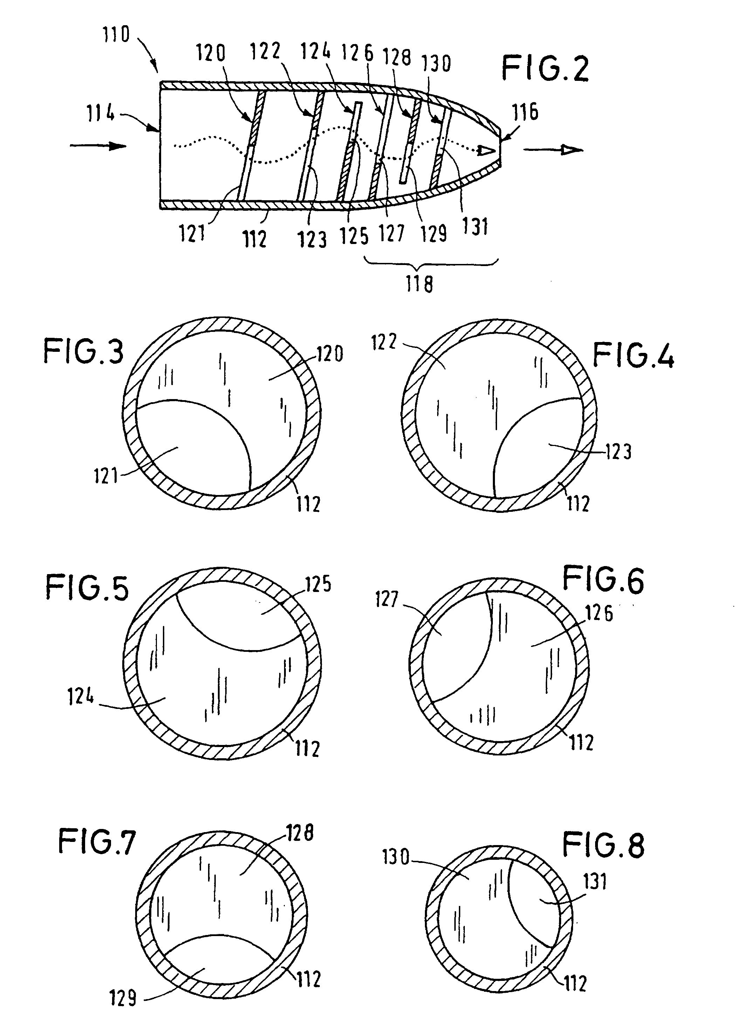

Fig. 2 is a perspective view of a first embodiment of a particle separator 110. The particle separator 110 comprises a substantially cylindrical housing 112 having an inlet opening 114 on one end and an outlet opening 116 on the other end. In its end portion 118 formed with the outlet opening 116, housing 112 is of a conically tapered shape.

Housing 112 can be mounted with its inlet opening 114 e.g. onto the central housing portion 16 of inhalator 10. Outlet opening 116 of housing 112 is adapted to have a mouthpiece connected thereto; alternatively, the end of housing 112 comprising the outlet opening 116 can be formed as a mouthpiece.

As evident from Fig. 2, housing 112 has arranged therein a plurality (in the instant embodiment, six) of partition walls 120 to 130. The distance between adjacent partition walls decreases from inlet opening 114 to outlet opening 116. Each of the partition walls 120 to 130 is provided with an opening 121 to 131 formed as an edge recess in partition walls 120 to 130. The size of the openings 121 to 131 decreases from inlet opening 114 to outlet opening 116 of housing 112 (cf. particularly Figs. 3 to 8). The openings 121 to 131 are arranged at positions rotated relative to each other (e.g. by 75° to 110°) about the longitudinal axis of particle separator 30. In this manner, the flow of the carrier gas and the particles passing through housing 112 is given a helical shape whose windings become narrower towards outlet opening 116 and whose flow velocity becomes larger towards outlet opening 116. Thereby, the heavier and larger particles are fractionated so that only the lighter, smaller particles will remain in the carrier gas flow, this effect being largely independent from the individual air flow rate of the patient which, as a matter of experience, is between 15 dm³ and 90 dm³ per minute.

All of the partition walls 120-130 are inclined into the same direction, notably at an angle of substantially 75° to 80° relative to the longitudinal extension of housing 112 between the inlet 114 and the oulet 116.

Fig. 9 shows an alternative embodiment of the particle separator 110', wherein components identical to those in Fig. 2 are provided with the same reference numerals in Fig. 9. Different from Fig. 2, the partition walls 120-130 in the particle separator 110' of Fig. 9 are inclined alternately in different directions in a manner resulting in a zig-zag-like configuration of the partition walls.

A further alternative embodiment of the particle separator 110" comprises partition walls 120-130 which are inclined about different axes of inclination, i.e. are inclined and turned in three dimensions in a plurality of different directions in the manner of swash plates.

## Claims

1. A particle separator for separating a particle fraction of an inhalable particle/air flow generated by inspired air, said particle separator being provided particularly to be mounted on a device for generating inhalable particles by release of said particles from a preferably solidified medicant supply, comprising
- a housing (18;112) having said particle/air flow (56) flowing therethrough, said housing (18;112) comprising an inlet (44;114) and an outlet (46;116), and
- a plurality of partition walls (120-130) arranged between said inlet (114) and said outlet (116) behind each other in the direction of the particle/air flow (56),
- each partition wall (120-130) having at least one opening (121-131) formed therein, and
- the openings (121-131) of adjacent partition walls (120-130) being arranged in a mutually displaced relationship resulting in a curvilinear portion of the flow path,
**characterized in**
- **that** the partition walls (120-130) are inclined at an angle of 65° to 85°, particularly 75° to 80°, relative to the extension of the housing (112) between the inlet (114) and the outlet (116) of the housing (112).

2. The particle separator according to claim 1, **characterized in that** the partition walls (120-130) are arranged in parallel to each other.

3. The particle separator according to claim 1 or 2, **characterized in that** the partition walls (120-130) can be divided into at least two groups and within each group are respectively arranged in parallel, the angles of inclination of the partition walls (120-130) of the two groups being different, particularly inverse to each other.

4. The particle separator according to claim 3, **characterized in that** the successive partition walls (120-130) have different angles of inclination following each other alternately.

5. The particle separator according to claim 3, **characterized in that** at least three partition walls (120-130) are provided.

6. The particle separator according to claim 1 or 2, **characterized in that** up to twenty, particularly up to ten and preferably up to seven partition walls (120-130) are provided.

7. The particle separator according to any one of claims 1 to 6, **characterized in that** the size of the openings (121-131) of the partition walls (120-130) is different from partition wall to partition wall.

8. The particle separator according to claim 7, **characterized in that** the openings (121-131) of the partition walls (120-130) decrease in size, particularly in a uniform manner, from the inlet (114) to the outlet (116) of the housing (112).

9. The particle separator according to any one of claims 1 to 8, **characterized in that** the distance between respectively adjacent partition walls (120-130) is different from partition wall to partition wall.

10. The particle separator according to claim 9, **characterized in that** the distance between respectively adjacent partition walls (120-130) decreases, particularly in a uniform manner, from the inlet (114) to the outlet (116).

11. The particle separator according to any one of claims 1 to 10, **characterized in that** the openings (121-131) are formed by in particular round edge recesses of the partition walls (120-130), each of the openings (121-131) in a partial region thereof being delimited by the housing (112).

12. The particle separator according to any one of claims 1 to 11, **characterized in that** the housing (112) is tapered, particularly conically, towards the outlet (116) to form a mouthpiece.

13. The particle separator according to any one of claims 1 to 12, **characterized in that** the openings (121-131) of the partition walls (120-130) are arranged at positions rotated relative to each other.

14. A device for generating an inhalable active-substance-particle/air flow generated by inspired air, comprising
- a housing (12;14,16,18) having said particle/ air flow flowing therethrough along a flow path, said housing (12; 14,16,18) comprising an air flow inlet (50) and an active-substance particle/air flow outlet (46),
- a release device (20) for the release of active-substance particles from at least one partial surface of a supply of a medicant (26), said partial surface being arranged in the flow path behind said air flow inlet (50), and
- a particle separator (30) arranged in the housing (12;14,16,18) for separating particles, substantially active-substance particles, which are larger than a maximum size, said particle separator (30) being designed according to any one of the preceding claims.

## Patentansprüche

1. Partikelabscheider zum Abscheiden einer Partikelfraktion eines inhalierfähigen, durch Einatmen von Luft aufgebauten Partikel/Luftstromes, insbesondere zum Aufsetzen auf eine Vorrichtung zur Erzeugung inhalierbarer Partikel durch Abtragen von einem vorzugsweise verfestigten Arzneimittelvorrat, mit
einem Gehäuse (18;112), das von dem Partikel-/Luftstrom (56) durchströmt wird, wobei das Gehäuse (18;112) einen Einlass (44;114) und einen Auslass (46; 116) aufweist, und
mehreren Trennwänden (120-130), die zwischen dem Einlass (114) und dem Auslass (116) in Richtung der Partikel-/Luftströmung (56) hintereinanderliegend angeordnet sind,
wobei jede Trennwand (120-130) mindestens eine Öffnung (121-131) aufweist und
wobei die Öffnungen (121-131) benachbarter Trennwände (120-130) zur Erzeugung eines krummlinienförmigen Strömungswegabschnitts gegeneinander versetzt angeordnet sind,
**dadurch gekennzeichnet,**
**dass** die Trennwände (120-130) unter einem Winkel von 65° bis 85°, insbesondere 75° bis 80°, zur Erstreckung des Gehäuses (112) zwischen dessen Einlass (114) und Auslass (116) geneigt angeordnet sind.

2. Partikelabscheider nach Anspruch 1, **dadurch gekennzeichnet, dass** die Trennwände (120-130) parallel zueinander angeordnet sind.

3. Partikelabscheider nach Anspruch 1 oder 2, **dadurch gekennzeichnet, dass** die Trennwände (120-130) sich in mindestens zwei Gruppen unterteilen lassen, innerhalb derer sie jeweils parallel angeordnet sind, wobei der Neigungswinkel der Trennwände (120-130) beider Gruppen unterschiedlich, insbesondere invers zueinander ist.

4. Partikelabscheider nach Anspruch 3, **dadurch gekennzeichnet, dass** die aufeinanderfolgenden Trennwände (120-130) abwechselnd unterschiedliche Neigungswinkel aufweisen.

5. Partikelabscheider nach Anspruch 3, **dadurch gekennzeichnet, dass** mindestens drei Trennwände (120-130) vorgesehen sind.

6. Partikelabscheider nach Anspruch 1 oder 2, **dadurch gekennzeichnet, dass** bis zu zwanzig, insbesondere bis zu zehn und vorzugsweise bis zu sieben Trennwände (120-130) vorgesehen sind.

7. Partikelabscheider nach einem der Ansprüche 1 bis 6, **dadurch gekennzeichnet, dass** die Größe der Öffnungen (121-131) der Trennwände (120-130) von Trennwand zu Trennwand unterschiedlich ist.

8. Partikalabscheider nach Anspruch 7, **dadurch gekennzeichnet, dass** sich die Öffnungen (121-131) der Trennwände (120-130) vom Einlass (114) bis zum Auslass (116) des Gehäuses (112) hin insbesondere gleichmäßig verkleinem.

9. Partikelabscheider nach einem der Ansprüche 1 bis 8, **dadurch gekennzeichnet, dass** der Abstand jeweils benachbarter Trennwände (120-130) von Trennwand zu Trennwand unterschiedlich ist.

10. Partikelabscheider nach Anspruch 9, **dadurch gekennzeichnet, dass** sich der Abstand jeweils benachbarter Trennwände (120-130) vom Einlass (114) bis zum Auslass (116) hin insbesondere gleichmäßig verkleinert.

11. Partikelabscheider nach einem der Ansprüche 1 bis 10, **dadurch gekennzeichnet, dass** die Öffnungen (121-131) durch insbesondere runde Randausnehmungen der Trennwände (120-130) gebildet sind, wobei jede Öffnung (121-131) in einem Teilbereich durch das Gehäuse (112) begrenzt ist.

12. Partikelabscheider nach einem der Ansprüche 1 bis 11, **dadurch gekennzeichnet, dass** sich das Gehäuse (112) zur Bildung eines Mundstücks (48) zum Auslass (116) hin insbesondere konisch verjüngt.

13. Partikelabscheider nach einem der Ansprüche 1 bis 12, **dadurch gekennzeichnet, dass** die Öffnungen (121-131) der Trennwände (120-130) gegeneinander verdreht angeordnet sind.

14. Vorrichtung zum Erzeugen eines inhalierbaren, durch Einatmen von Luft aufgebauten Wirkstoffpartikel-/Luftstroms mit
einem Gehäuse (12; 14,16,18), das von dem Partikel/Luftstrom entlang eines Strömungsweges durchströmt wird, wobei das Gehäuse (12; 14,16,18) einen Luftstromeinlass (50) und einen Wirkstoffpartikel-/Luftstromauslass (46) aufweist,
einer Abtragvorrichtung (20) zum Freisetzen von Wirkstoffpartikeln von zumindest einer im Strömungsweg hinter dem Luftstromeinlass (50) angeordneten Teilobertläche eines Arzneimittelvorrates (26) und
einem im Gehäuse (12;14,16,18) angeordneten Partikelabscheider (30) zum Abscheiden von im wesentlichen Wirkstoffpartikeln, die größer sind als eine Maximalgröße, wobei der Partikelabscheider (30) gemäß einem der vorhergehenden Ansprüche ausgebildet ist.

## Revendications

1. Séparateur de particules destiné à séparer une fraction de particules d'un écoulement particules/air inhalable produit par de l'air inspiré, ledit séparateur de particules étant prévu en particulier pour être monté sur un dispositif pour produire des particules inhalables par libération desdites particules à partir d'une réserve de médicament, de préférence à l'état solide, comprenant
- un boîtier (18 ; 112) traversé par l'écoulement dudit écoulement particules/air (56), ledit boîtier (18 ; 112) comprenant une entrée (44 ; 114) et une sortie (46 ; 116), et
une pluralité de parois de séparation (120 à 130) agencées entre ladite entrée (114) et ladite sortie (116), les unes derrière les autres, dans la direction de l'écoulement particules/air (56),
chaque paroi de séparation (120 à 130) comportant au moins une ouverture (121 à 131) formée dans celle-ci, et
les ouvertures (121 à 131) des parois de séparation adjacentes (120 à 130) étant agencées selon une relation mutuellement séparée produisant une partie curviligne du trajet d'écoulement,
**caractérisé en ce que**
les parois de séparation (120 à 130) sont inclinées d'un angle de 65° à 85°, en particulier de 75° à 80°, par rapport à l'extension du boîtier (112) entre l'entrée (114) et la sortie (116) du boîtier (112).

2. Séparateur de particules selon la revendication 1, **caractérisé en ce que** les parois de séparation (120 à 130) sont agencées en parallèle entre elles.

3. Séparateur de particules selon la revendication 1 ou 2, **caractérisé en ce que** les parois de séparation (120 à 130) peuvent être divisées au moins en deux groupes, et à l'intérieur de chaque groupe, sont respectivement agencées en parallèle, les angles d'inclinaison des parois de séparation (120 à 130) des deux groupes étant différents, en particulier, inverses les uns des autres.

4. Séparateur de particules selon la revendication 3, **caractérisé en ce que** les parois de séparation successives (120 à 130) ont des angles d'inclinaison différents en se suivant alternativement les unes les autres.

5. Séparateur de particules selon la revendication 3, **caractérisé en ce qu'**au moins trois parois de séparation (120 à 130) sont prévues.

6. Séparateur de particules selon la revendication 1 ou 2, **caractérisé en ce que** jusqu'à vingt, en particulier jusqu'à dix et de préférence jusqu'à sept parois de séparation (120 à 130) sont prévues.

7. Séparateur de particules selon l'une quelconque des revendications 1 à 6, **caractérisé en ce que** la taille des ouvertures (121 à 131) des parois de séparation (120 à 130) est différente d'une paroi de séparation à une autre.

8. Séparateur de particules selon la revendication 7, **caractérisé en ce que** la taille des ouvertures (121 à 131) des parois de séparation (120 à 130) diminue, en particulier d'une manière uniforme, de l'entrée (114) à la sortie (116) du boîtier (112).

9. Séparateur de particules selon l'une quelconque des revendications 1 à 8, **caractérisé en ce que** la distance entre des parois de séparation adjacentes (120 à 130) est différente d'une paroi de séparation à une autre.

10. Séparateur de particules selon la revendication 9, **caractérisé en ce que** la distance entre des parois de séparation respectivement adjacentes (120 à 130) diminue, en particulier d'une manière uniforme, de l'entrée (114) à la sortie (116).

11. Séparateur de particules selon l'une quelconque des revendications 1 à 10, **caractérisé en ce que** les ouvertures (121 à 131) sont formées, en particulier par des cavités à bords arrondis des parois de séparation (120 à 130), chacune des ouvertures (121 à 131) dans une région partielle de celles-ci étant délimitée par le boîtier (112).

12. Séparateur de particules selon l'une quelconque des revendications 1 à 11, **caractérisé en ce que** le boîtier (112) est effilé, en particulier conique, vers la sortie (116), de façon à constituer une embouchure.

13. Séparateur de particules selon l'une quelconque des revendications 1 à 12, **caractérisé en ce que** les ouvertures (121 à 131) des parois de séparation (120 à 130) sont agencées dans des positions ayant fait l'objet d'une rotation les unes par rapport aux autres.

14. Dispositif pour produire un écoulement particules de substance active/air inhalable, produit par de l'air inspiré, comprenant
- un boîtier (12 ; 14, 16, 18) traversé par l'écoulement dudit écoulement particules de substance active/air (56), ledit boîtier (12 ; 14, 16, 18) comprenant une entrée d'écoulement d'air (50) et une sortie d'écoulement particules de substance active/air (46),
- un dispositif de libération (20) pour la libération des particules de substance active depuis au moins une surface partielle d'une réserve de médicament (26), ladite surface partielle étant agencée dans le trajet d'écoulement derrière ladite entrée d'écoulement d'air (50), et
- un séparateur de particules (30) agencé dans le boîtier (12 ; 14, 16, 18) pour séparer des particules, substantiellement des particules de substance active, qui sont plus grandes qu'une taille maximale, ledit séparateur de particules (30) étant conçu selon l'une quelconque des revendications précédentes.
